# EUROPEAN PATENT SPECIFICATION

(11) **EP 2 943 125 B1**
(45) Date of publication and mention of the grant of the patent: **19.09.2018**
(21) Application number: 14700523.5
(22) Date of filing: 06.01.2014
(51) Int. Cl.: A61B 6/04, A61B 6/00

(54) **MAMMOGRAPHY APPARATUS WITH PRESSURE SENSORS AND METHOD, TO MONITOR AND ADJUST THE PRESSURE DISTRIBUTION**
MAMMOGRAPHIEGERÄT MIT DRUCKSENSOREN UND METHODE, ZUR ÜBERWACHUNG UND EINSTELLUNG DER DRUCK VERTEILUNG
APPAREIL DE MAMMOGRAPHIE AVEC CAPTEUR DE PRESSEUR ET PROCÉDÉ, POUR LA SURVEILLANCE ET L'AJUSTEMENT DE LA DISTRIBUTION DE LA PRESSION

(30) Priority: 08.01.2013 NL 2010095
(43) Date of publication of application: 18.11.2015
(73) Proprietor: Academisch Medisch Centrum bij de Universiteit van Amsterdam, 1105 AZ Amsterdam (NL)
(72) Inventor: GRIMBERGEN, Cornelis Antonius, 1105 AZ Amsterdam (NL); DEN HEETEN, Gerardus Johannes, 1105 AZ Amsterdam (NL)
(74) Representative: Van Breda, Jacobus
(86) International application number: PCT/NL2014/050001
(87) International publication number: WO 2014/109636

(56) References cited:
- DE-A1-102006 038 163
- JP-A- H08 299 329
- JP-A- 2009 285 345
- US-A- 5 506 877
- US-A1- 2008 080 668
- US-A1- 2008 181 361
- US-A1- 2008 240 346
- US-A1- 2009 262 887
- US-A1- 2012 020 464

## Description

The invention relates to a mammography apparatus comprising a mainframe and a sub-frame mounted in the mainframe, which sub-frame supports an x-ray source, a paddle and a detector with a detector cover, and which paddle is connected with the sub-frame through a suspension. The suspension of the paddle comprises force sensors for providing measurement signal that is indicative of an amount of compression applied with the paddle to a breast. The inclination of the paddle is controlled by a control unit and depending on the measurement signals from the force sensors.

The invention further relates to a method to monitor and/or adjust the settings of such a mammography apparatus.

A mammography apparatus is known from WO2011/102713 in the name of the applicant.

In such a known mammography apparatus it is day to day practice to place and compress a patient's breast between the detector and the paddle before executing an x-ray measurement in order to optimize the image quality and minimize the required x-ray doses. During the procedure the patient is standing next to the mammograph. The compression is applied by bringing the patient's breast in between the x-ray detector and the paddle, which paddle can thereafter be lowered in the direction and relative to the detector by hand or by activating a motor built in the mammography apparatus. Generally speaking there is a minimum pressure required to provide sufficient flattening to the breast to suit the requirements of the x-ray imaging. For safety reasons there is a maximum amount of force that can be applied which is set at a value of 20 daN. Consequently a wide variety of pressures can be applied depending on the contact area of the compressed breast on which the force is exercised. For that reason the upper limit of compression that can be applied depends on the size of the women's breasts. It is commonly known that this procedure may inflict serious pain on the patient. Also bruises and hematomas are reported on a regular base. To alleviate these problems it is important that the amount of pressure applied to the breast remains restricted. There are, however, situations during examination that may require that the pressure applied to the breast is increased in order to meet the required x-ray imaging conditions. This may for instance occur during a cranio caudal examination of the breast when a skin crease or stretch of the skin reduces the effectivity of the applied pressure. It may also occur during a medial lateral oblique examination of the breast when the paddle pressure is applied in part to the shoulder area and/or the breast muscle. Also this adversely affects the effective pressure applied to the breast. In the prior art these problems are addressed by increasing the pressure applied to the breast, therewith worsening the pain sensations and inflicting other adverse effects for the patient.

It is one of the objectives of the invention to improve the effectivity of the pressure applied to the breast.

It is a further objective of the invention to reduce the patient's experience of pain.

It is another objective of the invention to improve the reliability and repeatability of the x-ray measurement by providing a more reliable and reproducible compression of the breast.

It is still another objective of the invention to provide an improved mammography apparatus which ensures that the objectives of an optimal trade-off between reduced pain and more reliable and repeatable x-ray measurements can be achieved.

These and other objectives of the invention which will become apparent from the following disclosure, are attained with the method and mammography apparatus according to one or more of the appended claims.

According to US2012/0020464 a mammography apparatus according to the preamble is taught, wherein the suspension of the paddle comprises force sensors for providing a measurement signal that is indicative of an amount of compression applied with the paddle to a breast. Providing the force sensors in the suspension of the paddle ensures that a very accurate and reliable measurement of the compression applied to the breast can be obtained.

US5506877 and US2008/0181361 disclose a mammography apparatus wherein the inclination of a paddle is controlled by a single force sensor.

According to the invention, a mammography apparatus is provided with a measurement and control unit to actuate a drive motor for controlling an inclination of the paddle with reference to the detector cover depending on the measurement signals from the force sensors, wherein the measurement and control unit is arranged to actuate the drive motor for controlling an inclination of the paddle with reference to the detector cover so as to equalize the forces as measured with the respective force sensors. This provides the best estimate for a uniform distribution of forces along the breast during compression and imaging, can provide important diagnostic information and can be helpful in mitigating the pain that the patient experiences to the least possible extent.

Preferably there is at least a first force sensor and a second force sensor, that are placed at mirror image positions with reference to the sub-frame. This makes the measurement possible of the forces that are applied to the left and right side of the patient's breast.

The force sensors are advantageously placed at a position to prevent interference with the X-ray beam. This makes possible that a force exerted with the paddle during pressurizing of a breast is measured with said force measurement means during pressurizing and imaging so as to enable balancing or equalizing the force exerted on the contact area between the paddle and the breast during the imaging process.

The balancing of the measured forces can be done manually or automatically, in the latter case for instance using springs or a motor drive.

Particularly when manual adjustment of the mammography apparatus is carried out, it is advantageous that the measurement and control unit is arranged to indicate a differential force of the first force sensor and the second force sensor that are placed on mirror image positions with respect to the sub-frame. This may of course also be applied when the balancing of the measured forces is carried out automatically.

As mentioned the measurement and control unit is arranged to actuate a drive motor for controlling an inclination of the paddle with reference to the detector cover depending on the measurements with the force sensors provided at mirror image positions with respect to the sub-frame so as to automatically equalize the forces as measured with these respective force sensors. This adjustment of the inclination of the paddle with reference to the detector cover will be limited to only a few degrees to maintain image quality, but enough to largely circumvent the influence of the earlier mentioned skin crease or the shoulder or breast muscle. This may apply both to a cranio caudal examination and with a medial lateral oblique examination of the breast.

Furthermore the distribution of forces as measured with the force sensors during compression may indicate important differences in tissue stiffness, which can indicate a mass which can potentially be invisible in the mammographic image. If the differential force between the forces measured by the first force sensor and by the second force sensor exceeds a predefined value, preferably an indicator signal is provided to warn an operator of the mammography apparatus that repositioning of the breast is indicated. If the disbalance persists the information can be transferred to the radiologist. The disbalance can for instance be expressed as the ratio between the measured forces at or near the respective sides of the paddle. The warning signal is particularly useful to indicate the operator that on one side of the paddle the compression is less or not useful and the desired goal of the compression i.e. flattening and immobilization of the breast during exposure cannot be achieved, and repositioning of the breast should be considered.

The invention will hereinafter be further elucidated with reference to the drawing.

In the drawing:
- figure 1 shows schematically a cranio caudal breast examination;
- figure 2 shows schematically a medial lateral oblique examination of the breast;
- figure 3A and 3B show schematically a frontal view and a side view respectively, of a mammography apparatus according to the invention;
- figure 4 shows a first embodiment of the mammography apparatus of the invention wherein the force sensors are applied at the back rods of the suspension; and
- figure 5 shows a second embodiment of the mammography apparatus of the invention wherein the force sensors are applied at the side rods of the suspension.

Whenever in the figures the same reference numerals are applied, these numerals refer to the same parts.

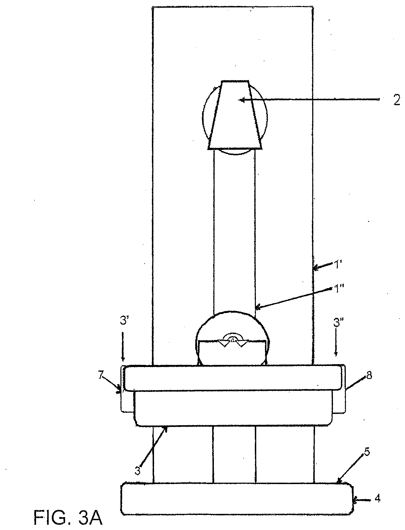

With reference first to figure 3A a frontal view is shown of a mammography apparatus 1 which has a mainframe 1' that is provided with an x-ray source 2, a paddle 3 and a detector 4 with a detector cover 5 that during use contacts the underside of a patient's breast. The person skilled in the art is aware that the x-ray source 2, the paddle 3 and the detector 4 are placed on a sub-frame 1" (also called c-arm) that is mounted in the mainframe 1'. The sub-frame 1" may be moved up and down and is rotatable.

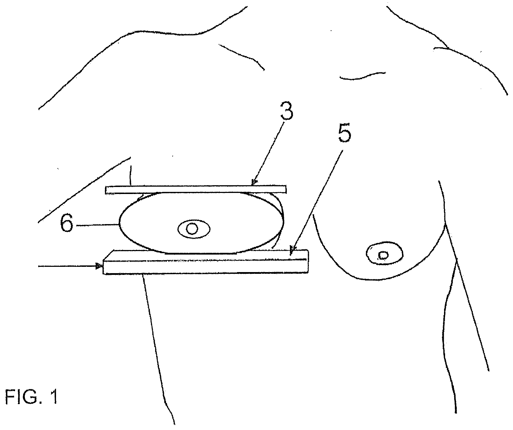

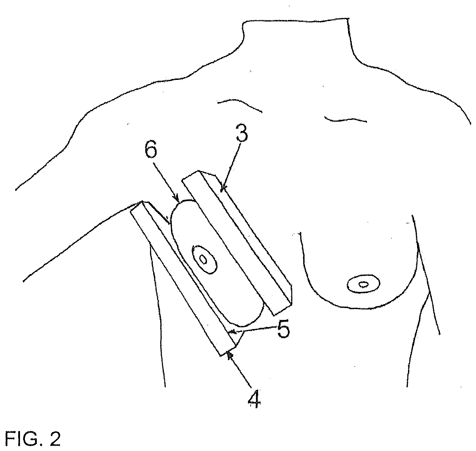

With reference to figure 1 a cranio caudal examination of a patient's breast 6 is shown, wherein for clarity only the paddle 3, the detector 4 and the detector cover 5 are shown with reference to said breast 6. In figure 2 the so-called medial lateral oblique examination of the breast 6 is shown, which is featured by the oblique positioning of the sub-frame 1" with reference to the frame 1' to cause that the paddle 3 and the detector 4 with the detector cover 5 are placed at an angle with reference to the horizontal.

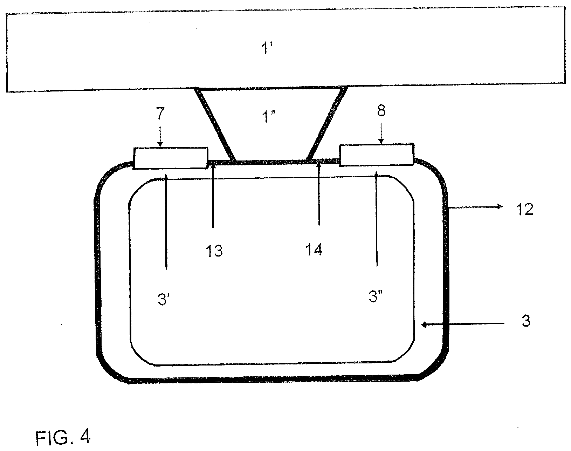

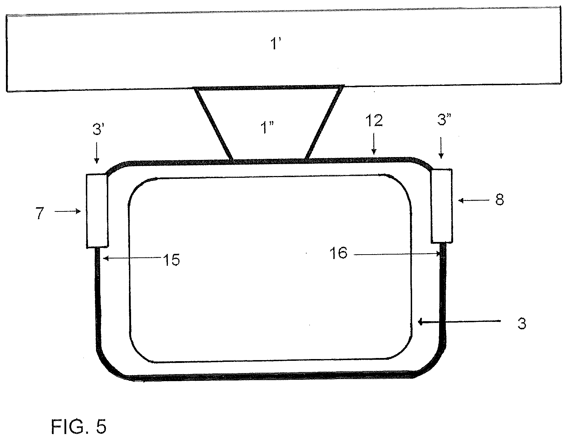

Turning again to figure 3A and also making reference to figure 4 and 5 it is shown that the paddle 3 has measurement means embodied as force sensors 7, 8 that are provided at positions 3', 3" as part of the suspension 12 of the paddle 3 and because thereof outside the X-ray beam originating from x-ray source 2. In figure 4 two force sensors 7, 8 are shown positioned in the back rods 13, 14 of the suspension 12; in figure 5 two force sensors 7, 8 are shown positioned in the side rods 15, 16 of the suspension 12. Both embodiments of figure 4 and figure 5 enable that the force that is exerted with the paddle 3 during pressurizing of a breast 6 is measured with said force sensors 7, 8 so that these respective forces at both sides of the paddle 3 can be balanced or equalized.

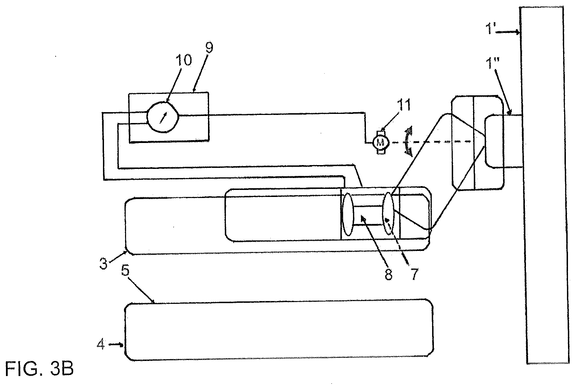

Figure 3B further shows that the mammography apparatus 1 according to the shown preferred embodiment is further provided with a measurement and control unit 9 that is connected to the first force sensor 7 which is placed at a first position 3' in the suspension 12 of the paddle 3 and to the second force sensor 8 which is placed at a second position 3' in the suspension 12 of the paddle 3. The two said positions 3', 3" are mirror image positions with reference to the sub-frame 1". The measurement and control unit 9 is further arranged to indicate, preferably with indicator means 10, a differential force of the first force sensor 7 and the second force sensor 8. This can then be suitably used to actuate the paddle either manually or automatically depending on the measurements with the force sensors 7, 8 provided at the mirror image positions 3', 3" in the suspension 12 of the paddle 3.

Figure 3B shows that the measurement and control unit 9 is arranged to actuate a drive motor 11 for controlling an inclination of the paddle 3 with reference to the detector cover 5 depending on the measurements with the force sensors 7, 8 provided at the mirror image positions 3', 3" in the suspension 12 of the paddle 3 so as to equalize the forces as measured with these respective force measurement means 7, 8.

It is emphasized that the invention should not be deemed restricted to the exemplary embodiment of a mammography apparatus according to the invention with reference to the drawing. This exemplary embodiment merely serves to elucidate the wording as used in the appended claims. The protective scope that merits the invention is, however, exclusively determined by the appended claims, wherein the meaning of the wording used in the claims can be explained with reference to the description and this exemplary embodiment.

## Claims

1. Mammography apparatus (1) comprising a mainframe (1') and a sub-frame (1") mounted in the mainframe (1'), which sub-frame (1") supports an x-ray source (2), a paddle (3) and a detector (4) with a detector cover (5), and which paddle (3) is connected with the sub-frame (1") through a suspension (12), wherein the suspension (12) of the paddle (3) comprises force sensors (7, 8) for providing measurement signals that are indicative of an amount of compression applied with the paddle (3) to a breast, **characterized in that** a measurement and control unit (9) is provided to actuate a drive motor (11) for controlling an inclination of the paddle (3) with reference to the detector cover (5) depending on the measurement signals from the force sensors (7, 8), wherein the measurement and control unit (9) is arranged to actuate the drive motor (11) for controlling an inclination of the paddle (3) with reference to the detector cover (5) so as to substantially equalize the forces as measured with the respective force sensors (7, 8).

2. Mammography apparatus (1) according to claim 1, **characterized in that** there is at least a first force sensor (7) and a second force sensor (8), that are placed at mirror image positions (3', 3") with reference to the sub-frame (1").

3. Mammography apparatus (1) according to claim 1 or 2, **characterized in that** the force sensors (7, 8) are placed at a position (3', 3") within the suspension (12) of the paddle (3) to prevent interference with an x-ray beam from the x-ray source.

4. Mammography apparatus (1) according to any one of the previous claims 1-3, **characterized in that** the measurement and control unit (9) is arranged to indicate a differential force of the first force sensor (7) and the second force sensor (8).

5. Method for monitoring and/or adjusting the settings of a mammography apparatus (1) comprising a mainframe (1'), a sub-frame (1") mounted in the mainframe (1'), wherein the sub-frame (1") supports an x-ray source (2), a paddle (3) and a detector (4) with a detector cover (5), including the step of providing a suspension (12) for connecting the paddle (3) with the sub-frame (1"), and the step of providing the suspension (12) of the paddle (3) with force sensors (7, 8) for obtaining a measurement signal that is indicative of an amount of compression applied with the paddle (3) to a breast, **characterized by** providing a drive motor (11) for controlling an inclination of the paddle (3) with reference to the detector cover (5) depending on the measurements with the force sensors (7, 8), wherein the drive motor (11) is actuated for controlling an inclination of the paddle (3) with reference to the detector cover (5) depending on the measurements with the force sensors (7, 8) so as to equalize the forces as measured with these respective force sensors (7, 8).

6. Method according to claim 5, **characterized by** providing the force sensors (7, 8) within the suspension (12) of the paddle (3) on mirror image positions (3', 3") with reference to the sub-frame (1").

7. Method according to claim 5 or 6, **characterized by** providing the force sensors (7, 8) at a position (3', 3") within the suspension (12) of the paddle (3) to prevent interference with an x-ray beam from the x-ray source.

8. Method according to any one of the previous claims 5-7, **characterized by** providing that the forces measured with the respective force sensors (7, 8) are compared to indicate a differential force between said forces measured by the force sensors (7, 8).

9. Method according to any one of claims 5-8, **characterized by** providing that the paddle (3) is actuated manually or automatically depending on the measurements with the force sensors (7, 8).

10. Method according to any one of the previous claims 5-9, **characterized by** providing an indicator signal to warn an operator of the mammography apparatus (1) that repositioning of the breast is indicated if a differential force between the forces measured by the force sensors (7, 8) that are placed on mirror image positions (3', 3") with respect to the sub-frame (1"), exceeds a predefined value.

## Patentansprüche

1. Mammographiegerät (1) mit einem Hauptrahmen (1') und einem im Hauptrahmen (1') montierten Hilfsrahmen (1"), wobei der Hilfsrahmen (1") eine Röntgenquelle (2), eine Platte (3) und einen Detektor (4) mit einer Detektorabdeckung (5) trägt, und wobei die Platte (3) mit dem Hilfsrahmen (1") durch eine Aufhängung (12) verbunden ist, wobei die Aufhängung (12) der Platte (3) Kraftsensoren (7, 8) zum Bereitstellen von Messsignalen umfasst, die einen mit der Platte (3) auf eine Brust ausgeübten Andruckwert anzeigen, **dadurch gekennzeichnet, dass** eine Mess- und Steuereinheit (9) vorgesehen ist, um einen Antriebsmotor (11) zum Steuern einer Neigung der Platte (3) in Bezug auf die Detektorabdeckung (5) in Abhängigkeit von den Messsignalen von den Kraftsensoren (7, 8) zu betätigen, wobei die Mess- und Steuereinheit (9) angeordnet ist, um den Antriebsmotor (11) zum Steuern einer Neigung der Platte (3) in Bezug auf die Detektorabdeckung (5) so zu betätigen, um die mit den jeweiligen Kraftsensoren (7, 8) gemessenen Kräfte im Wesentlichen auszugeichen.

2. Mammographiegerät (1) nach Anspruch 1, **dadurch gekennzeichnet, dass** wenigstens ein erster Kraftsensor (7) und ein zweiter Kraftsensor (8) vorhanden sind, die an Spiegelbildpositionen (3', 3") bezogen auf den Hilfsrahmen (1") angeordnet sind.

3. Mammographiegerät (1) nach Anspruch 1 oder 2, **dadurch gekennzeichnet, dass** die Kraftsensoren (7, 8) an einer Stelle (3', 3") innerhalb der Aufhängung (12) der Platte (3) angeordnet sind, um eine Interferenz mit einem Röntgenstrahl von der Röntgenquelle zu verhindern.

4. Mammographiegerät (1) nach einem der vorangehenden Ansprüche 1 bis 3, **dadurch gekennzeichnet, dass** die Mess- und Steuereinheit (9) zur Anzeige einer Differenzkraft des ersten Kraftsensors (7) und des zweiten Kraftsensors (8) angeordnet ist.

5. Verfahren zum Überwachen und/oder Einstellen der Einstellungen eines Mammographiegerätes (1) mit einem Hauptrahmen (1'), einem im Hauptrahmen (1') montierten Hilfsrahmen (1"), wobei der Hilfsrahmen (1") eine Röntgenquelle (2) trägt, eine Platte (3) und ein Detektor (4) mit einer Detektorabdeckung (5), umfassend den Schritt des Bereitstellens einer Aufhängung (12) zum Verbinden der Platte (3) mit dem Hilfsrahmen (1"), und den Schritt des Bereitstellens der Aufhängung (12) der Platte (3) mit Kraftsensoren (7, 8) zum Erhalten eines Messsignals, das einen mit der Platte (3) auf eine Brust ausgeübten Andruckwert anzeigt, **gekennzeichnet durch** Bereitstellen eines Antriebsmotors (11) zum Steuern einer Neigung der Platte (3) in Bezug auf die Detektorabdeckung (5) in Abhängigkeit von den Messungen mit den Kraftsensoren (7), 8), wobei der Antriebsmotor (11) zur Steuerung einer Neigung der Platte (3) bezüglich der Detektorabdeckung (5) in Abhängigkeit von den Messungen mit den Kraftsensoren (7, 8) betätigt wird, um die mit diesen jeweiligen Kraftsensoren (7, 8) gemessenen Kräfte auszugleichen.

6. Verfahren nach Anspruch 5, **gekennzeichnet durch** Bereitstellen der Kraftsensoren (7, 8) innerhalb der Aufhängung (12) der Platte (3) an Spiegelbildpositionen (3', 3") bezogen auf den Hilfsrahmen (1").

7. Verfahren nach Anspruch 5 oder 6, **gekennzeichnet durch** Bereitstellen der Kraftsensoren (7, 8) an einer Position (3', 3") innerhalb der Aufhängung (12) der Platte (3), um eine Interferenz mit einem Röntgenstrahl von der Röntgenquelle zu verhindern.

8. Verfahren nach einem der vorangehenden Ansprüche 5 bis 7, **gekennzeichnet durch** Bereitstellen, dass die mit den jeweiligen Kraftsensoren (7, 8) gemessenen Kräfte verglichen werden, um eine Differenzkraft zwischen den von den Kraftsensoren (7, 8) gemessenen Kräften anzuzeigen.

9. Verfahren nach einem der Ansprüche 5 bis 8, **gekennzeichnet durch** Bereitstellen, dass die Platte (3) in Abhängigkeit von den Messungen mit den Kraftsensoren (7, 8) manuell oder automatisch betätigt wird.

10. Verfahren nach einem der vorangehenden Ansprüche 5 bis 9, **gekennzeichnet durch** Bereitstellen eines Indikatorsignals, um einen Nutzer des Mammographiegerätes (1) zu warnen, dass ein Repositionieren der Brust indiziert ist, wenn eine Differenzkraft zwischen den von den Kraftsensoren (7, 8) gemessenen Kräften, die auf Spiegelbildpositionen (3', 3") in Bezug auf den Hilfsrahmen (1") angeordnet sind, einen vorbestimmten Wert überschreitet.

## Revendications

1. Appareil de mammographie (1), comprenant un bâti principal (1') et un sous-bâti (1") monté dans le bâti principal (1'), ce sous-bâti (1") supportant une source de rayons X (2), une palette (3) et un détecteur (4) avec un capot de détecteur (5), et cette palette (3) étant reliée au sous-bâti (1") par l'intermédiaire d'une suspension (12), la suspension (12) de la palette (3) comprenant des capteurs de force (7, 8) pour délivrer des signaux de mesure qui sont indicatifs d'une ampleur de compression appliquée avec la palette (3) à un sein, **caractérisé en ce qu'**une unité de mesure et de commande (9) est disposée de façon à actionner un moteur d'entraînement (11) pour commander une inclinaison de la palette (3) en référence au capot de détecteur (5) en fonction des signaux de mesure venant des capteurs de force (7, 8), dans lequel l'unité de mesure et de commande (9) est agencée de façon à actionner le moteur d'entraînement (11) de façon à commander une inclinaison de la palette (3) en référence au capot de détecteur (5) de façon à égaliser sensiblement les forces mesurées avec les capteurs de force respectifs (7, 8).

2. Appareil de mammographie (1) selon la revendication 1, **caractérisé en ce qu'**il y a au moins un premier capteur de force (7) et un deuxième capteur de force (8), qui sont disposés dans des positions d'image de miroir (3', 3") en référence au sous-bâti (1").

3. Appareil de mammographie (1) selon la revendication 1 ou 2, **caractérisé en ce que** les capteurs de force (7, 8) sont disposés dans une position (3', 3") à l'intérieur de la suspension (12) de la palette (3) pour empêcher une interférence avec un faisceau de rayons X venant de la source de rayons X.

4. Appareil de mammographie (1) selon l'une quelconque des revendications 1 à 3 qui précèdent, **caractérisé en ce que** l'unité de mesure et de commande (9) est agencée de façon à indiquer une force différentielle du premier capteur de force (7) et du deuxième capteur de force (8).

5. Procédé pour contrôler et/ou ajuster les réglages d'un appareil de mammographie (1) comprenant un bâti principal (1'), un sous-bâti (1") monté dans le bâti principal (1'), le sous-bâti (1") supportant une source de rayons X (2), une palette (3) et un détecteur (4) avec un capot de détecteur (5), comprenant l'étape de disposition d'une suspension (12) pour relier la palette (3) au sous-bâti (1"), et l'étape consistant à munir la suspension (12) de la palette (3) de capteurs de force (7, 8) de façon à obtenir un signal de mesure qui est indicatif d'une ampleur de compression appliquée avec la palette (3) à un sein, **caractérisé par** la disposition d'un moteur d'entraînement (11) pour commander une inclinaison de la palette (3) en référence au capot de détecteur (5) en fonction des mesures avec les capteurs de force (7, 8), dans lequel le moteur d'entraînement (11) est actionné de façon à commander une inclinaison de la palette (3) en référence au capot de détecteur (5) en fonction des mesures avec les capteurs de force (7, 8) de façon à égaliser les forces mesurées avec ces capteurs de force respectifs (7, 8).

6. Procédé selon la revendication 5, **caractérisé par** la disposition des capteurs de force (7, 8) à l'intérieur de la suspension (12) de la palette (3) sur des positions d'image de miroir (3', 3") en référence au sous-bâti (1").

7. Procédé selon la revendication 5 ou 6, **caractérisé par** la disposition des capteurs de force (7, 8) dans une position (3', 3") à l'intérieur de la suspension (12) de la palette (3) de façon à empêcher une interférence avec un faisceau de rayons X venant de la source de rayons X.

8. Procédé selon l'une quelconque des revendications 5 à 7 qui précèdent, **caractérisé en ce que** les forces mesurées avec les capteurs de force respectifs (7, 8) sont comparées de façon à indiquer une force différentielle entre lesdites forces mesurées par les capteurs de force (7, 8) .

9. Procédé selon l'une quelconque des revendications 5 à 8, **caractérisé en ce que** la palette (3) est actionnée manuellement ou automatiquement en fonction des mesures avec les capteurs de force (7, 8).

10. Procédé selon l'une quelconque des revendications 5 à 9 qui précèdent, **caractérisé par** la délivrance d'un signal d'indicateur de façon à avertir un opérateur de l'appareil de mammographie (1) du fait qu'un repositionnement du sein est indiqué si une force différentielle entre les forces mesurées par les capteurs de force (7, 8) qui sont disposés sur des positions d'image de miroir (3', 3") par rapport au sous-bâti (1") dépasse une valeur prédéfinie.
